# EUROPEAN PATENT APPLICATION

(11) **EP 4 083 182 A1**
(43) Date of publication of application: **02.11.2022**
(21) Application number: 21170536.3
(22) Date of filing: 26.04.2021
(51) Int. Cl.: C12M 1/12, A61L 2/28, C12M 1/34, C12M 1/36, C12Q 1/22

(54) **METHOD FOR STERILITY TESTING**

(71) Applicant: Sartorius Stedim Biotech GmbH, 37079 Göttingen (DE)
(72) Inventor: ARAKEL, Eric, Clement, Göttingen (DE); THENMAIER, Harald, Wien (AT); HIGGINBOTTON, Claire, Belleville MI (US); GUENEC, Olivier, Hamari (FI)
(74) Representative: Gottschald Patentanwälte Partnerschaft mbB

(57) **Abstract**

The invention relates to a method for sterility testing based on optically analyzing at least one test liquid (3), which test liquid (3) is contained in a liquid container (4), wherein depending on the contamination state of the test liquid (3), non liquid contaminants (5) are distributed in the test liquid. It is proposed, that in an analyzing routine (9) performed by a control arrangement (6), image-related data (10) representing at least one optical image (I) of the test liquid (3), generated by a sensor arrangement (11), are being transmitted from the sensor arrangement (11) to the control arrangement (6) and the contamination state of the test liquid (3) is derived from the image-related data (10) based on the interrelation between the distribution characteristics of the contaminants (5) and the respective contamination state.

## Description

The present invention relates to a method for sterility testing according to the general part of claim1, a data storage device with a training data set according to claim 24, a control arrangement according to claim 25, a sterility testing module according to claim 26 and a sterility testing system according to claim 27.

Sterility testing, for which this invention was designed, is of crucial importance for a wide range of biological and/or medical liquids. For example, in microbiological quality control testing, it has to be ensured that sterile preparations such as vaccines are free of viable contaminating microorganisms.

Today, sterility testing is performed in one of two ways: Direct inoculation, where a growth medium (called 'sample liquid') is inoculated with a test sample and incubated for a period prescribed by the pharamacopeia; or membrane- filtration. Membrane filtration involves filtering the test solution through a membrane filter enclosed within a closed system/ canister, filling it with a sample liquid and incubating it for a period prescribed by the pharmacopeia. After the incubation period, the sample liquid is visually inspected by an experienced laboratory professional. Any visual change, largely corresponding to turbidity, within the liquid, indicates the presence of contaminating microorganisms in the test sample.

The known method for sterility testing (US 7,354,758 B2), which is the starting point for the invention, is based on optically analyzing at least one test liquid, which is contained in a liquid container. This known sterility testing is performed by membrane filtration. This means that after the sample liquid, that is actually to be analyzed in view of sterility, has been passed through a membrane filter, a nutrient media in the form of a nutrient solution is introduced into the liquid container, which resulting liquid is called "test liquid" in the following. After an incubation period, the test liquid is visually inspected by an experienced laboratory professional. As the distribution of known contaminants in the test liquid in most cases goes along with a corresponding turbidity within the liquid, this optical inspection is a powerful measure to find out about the contamination state of the test liquid and as a result of the sample liquid. Based on the distribution characteristics of the contaminants in the test liquid it may also be possible to find out about the specific organism causing the contamination.

Despite the routine optimisation of accessories designed for sterility testing, such as containers, filters, pumps and connectors, the core method still largely relies on the experience of the respective laboratory professional for the visual inspection of the sample liquid. This systematically leads to a certain decrease in reproducibility, such that the known method for sterility testing is mostly conducted under the four-eyes-principle, which is cost- and time-consuming.

The problem underlying the invention is therefore to improve the known method for sterility testing such that the reproducibility is increased in a cost-effective and also time-effective way.

The above-noted problem is solved for a method for sterility testing with the features of the general part of claim 1 by the features of the characterizing part of claim 1.

It has been found that the interrelation between the distribution characteristics of the contaminants in the test liquid and the respective contamination state, which goes along with these distribution characteristics, may well be the basis for the automation of the examination of sterility testing. This is true because the distribution characteristics go along with characteristic turbidity within the fluid, which may easily be detected by a camera unit as optical contrasts, aggregation and/or sedimentation of particles, change in color and/or brightness or the like.

In detail, it is proposed that in an analyzing routine performed by a control arrangement, image-related data representing at least one optical image of the test liquid, generated by a sensor arrangement, are being transmitted from the sensor arrangement to the control arrangement. Within this analyzing routine, the contamination state of the test liquid is then derived from the image-related data based on the interrelation between the distribution characteristics of the contaminants in the test liquid and the respective contamination state.

With the proposed solution, the examination of sterility testing may now be performed in a fully automated and thereby fully reproducible way. The costs for realizing the proposed method are mainly software-related costs, while the hardware regarding the sensor arrangement and the control arrangement are comparably low.

The term "image-related data" is to be understood in a broad sense. It represents at least one image of the test liquid and can in this sense be a normal photographic representation. In addition, the term "image-related data" may comprise sensor data of other sensors of the sensor arrangement regarding other properties of the test liquid, that contribute information regarding the contamination state to be derived based on the image-related data. Such properties may regard, for example, carbon-source concentration, nitrogen-source concentration, amino acid concentration, growth factors concentration, pH, temperature, oxygen concentration, carbon dioxide concentration, conductivity, pressure, DNA concentration, protein concentration, biomass concentration, biomass production rate, oxygen uptake rate and/or metabolites, such as lactic acid.

In line with this understanding, the analyzing routine, which is relying on the image-related data, is also called "image-related analyzing routine". The image-related analyzing routine may in addition include taking into account sensor data of other sensors for the derivation of the contamination state.

The proposed method may be realized for a wide variety of sterility testing concepts. In this context, claim 2 points out sterility testing based on membrane filtration as a first preferred alternative and sterility testing based on direct inoculation as a second preferred alternative. Other alternatives are well applicable here.

According to claim 3, it is clarified, that the distribution of the contaminants, which detection is the basis for the proposed method, may be represented by different aspects of turbidity, each taken alone or in combination. Each of those aspects may well be detected by a camera unit or the like.

According to claims 4 to 7, prior property data are being generated in a prior analyzing routine, which is preceding the above noted, image-related analyzing routine. The term "preceding" means here, that the prior analyzing routine is performed, before the contamination state of the test liquid has been derived in the image-related analyzing routine. Accordingly, there may be a considerable overlapping taking place between those analyzing routines. The advantage is that it offers a higher level of reproducibility and reliability, since it allows for an earlier detection of potential contaminants, while simultaneously being as compliant as possible to official regulations, such as the pharmacopeia.

During the prior analyzing routine, properties of the test liquid may be obtained such as, again for example, carbon-source concentration, nitrogen-source concentration, amino acid concentration, growth factors concentration, pH, temperature, oxygen concentration, carbon dioxide concentration, conductivity, pressure, DNA concentration, protein concentration, biomass concentration, biomass production rate, oxygen uptake rate and/or metabolites, such as lactic acid.

The above may be advantageous in order to improve the reliability and/or the level of detail of the results of the image-related analyzing routine by additionally taking into acount the prior property data (claim 6). In addition or as an alternative, the prior analyzing routine may provide a rapid detection of the contamination state, which is called "preliminary contamination state" in the following (claim 7). The preliminary contamination state may later be confirmed by the image-related analyzing routine. It may be pointed out, that a number of prior analyzing routines may be part of the proposed method, that each work according to one or more of claims 4 to 7.

Claim 8 is directed to different definitions of the contamination state, each going back to representing the contamination state by a contamination class out of a predefined group of contamination classes. This means that the proposed method for sterility testing then goes back to classification based on the image-related data. Compared to a quantitative definition of the contamination state, such classification is easy to realize and, in addition, completely satisfactory in view of today's standards for sterility testing.

A first alternative for the realization of the proposed method according to claim 9 is simple image processing based on feature extraction, for example, wherein a combination of certain features like lines and formations may be assigned to the respective contamination class.

A second preferred alternative for the realization of the proposed method for sterility testing is subject of claims 10 to 15. Here it is proposed that the analyzing routine is based on a machine learning mechanism, which is trained to derive the contamination state from the image-related data and, if so, from the prior property data. This is a most powerful approach to achieve very good classification results even if the distribution characteristics of the contaminants varies with one and the same contaminating organism.

According to claim 12, preferably, the machine learning mechanism is based on a trained neural network, which is, further preferably, a neural convolution network (CNN). Such neural convolution networks have proven to be extremely effective for classification tasks based on images.

An effective way to reduce the necessary computation power for the classification step according to claim 14, it is preferred to define a region of interest within the respective image, which is subject to classification. This means, that not the whole image is being analyzed, but only the region of interest. This definition of the region of interest is done in a crop step, which may either be performed manually or automatically, for example, based on feature extraction.

Claim 15 is directed to a training step for training the machine learning mechanism, which training step is preferably based on annotated images representing the contamination classes to be derived in the analyzing routine. The result is a training data set, which is used by the machine learning mechanism in the classification step. The training step may be repeated at any time, such that the training data set may be continuously improved.

Claims 16 to 18 are directed to preferred variants for the realization of the sensor arrangement. According to claim 16, the sensor arrangement includes a camera unit, which is directed towards the test liquid. The camera unit may be a simple 2D camera unit, which has proven to be fully sufficient for a good classification result.

According to claim 17, the viewing direction of the camera unit is more or less parallel to the direction of gravity. In other words, the viewing direction of the camera unit is downwards or upwards with respect to gravity. In the preferred case, that the liquid container is a cup-like container, the camera is preferably viewing from above or from below the liquid container. It is easily understandable that this positioning of the camera unit makes it easily possible to analyze a large number of liquid containers, which are arranged side by side in a horizontal plane, as will be explained later.

The image-related data may preferably be directed not only to a single image of the test liquid but although to a series of at least two images of the test liquid, which is subject of claim 18.

In order to further improve the reproducibility of the proposed method, according to claim 19, it is proposed to provide the sensor arrangement with a light arrangement for illuminating the test liquid. Preferably, this light arrangement is being controlled by the control arrangement in order to modify the lighting properties.

Preferably, the proposed method is performed for at least two test liquids, which are each contained in a separate liquid container (claims 20, 21). According to claim 21, only one analyzing routine is performed for two or more test liquids, which are based on one and the same sample liquid. This is advantageous if the at least two test liquids include different nutrient solutions in order to improve the classification result for the sample liquid.

Claims 22 and 23 are directed to the application of a manipulation system in order to realize a relative movement between the liquid containers and at least part of the sensor arrangement. With this, it is possible to automatically align the respective part of the sensor arrangement, in particular the camera unit, with the respective liquid container, such that an automated examination of sterility testing of a large number of test liquids is possible.

According to another teaching of claim 24, which is of independent importance, a data storage device with a training data set for use in the proposed method is claimed as such. All explanations regarding the first teaching are fully applicable.

According to another teaching of claim 25, which is of independent importance as well, the control arrangement for performing the proposed method is claimed as such. Again, all explanations regarding the first teaching are fully applicable.

According to another teaching of claim 26, which is of independent importance as well, a sterility testing module comprising at least part of a sensor arrangement, in particular a camera unit, and at least part of a control arrangement is claimed as such, which sterility testing module is designed for performing the proposed method for sterility testing. Again all explanations given for the first teaching are equally applicable.

Another teaching according to claim 27, which is of independent importance as well, a sterility testing system with an above-noted sterility testing module and with a container carrier for carrying at least two liquid containers for test liquids is claimed as such. Again, all explanations given with regard to the first teaching are fully applicable.

Claims 28 to 30 are directed to providing the sterility testing system with a manipulation system and in particular, with a motorized manipulator, by which according to claim 30, the camera unit may be moved in the horizontal plane, preferably only in two dimensions. This is particularly advantageous if the camera unit is directed downwards or upwards with respect to gravity as explained with respect to claim 17. In addition or as an alternative, the camera unit may be moved in three dimensions.

In the following, an embodiment of the invention is explained with respect to the drawings. In the drawings show
- Fig. 1: a proposed sterility testing module of a sterility testing system during the analyzing routine,
- Fig. 2: a flowchart of the analyzing routine using a machine learning mechanism,
- Fig.3: a flowchart of the training step for the training of the machine learning mechanism, and
- Fig. 4: the sterility testing system with a sterility testing module of Fig. 1.

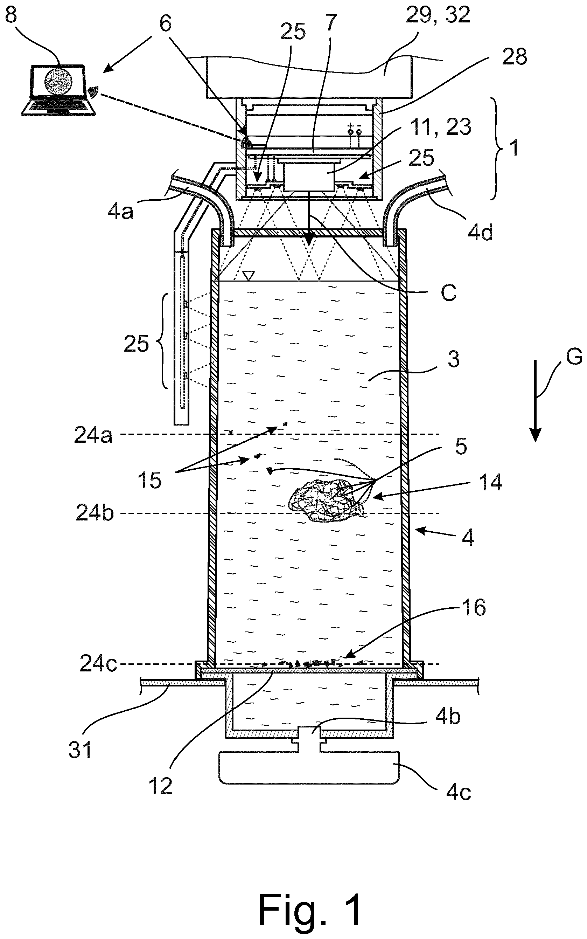

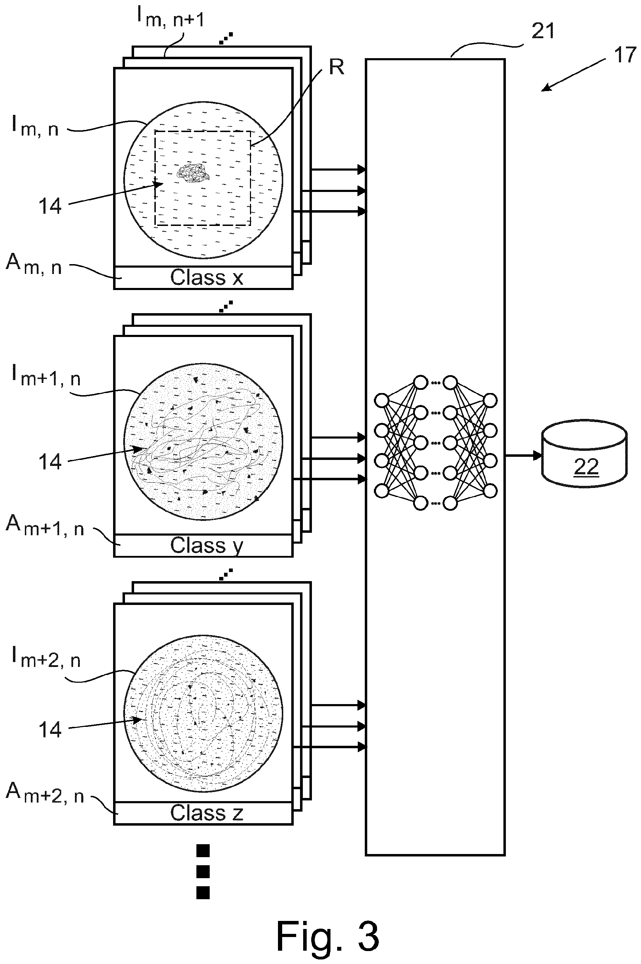

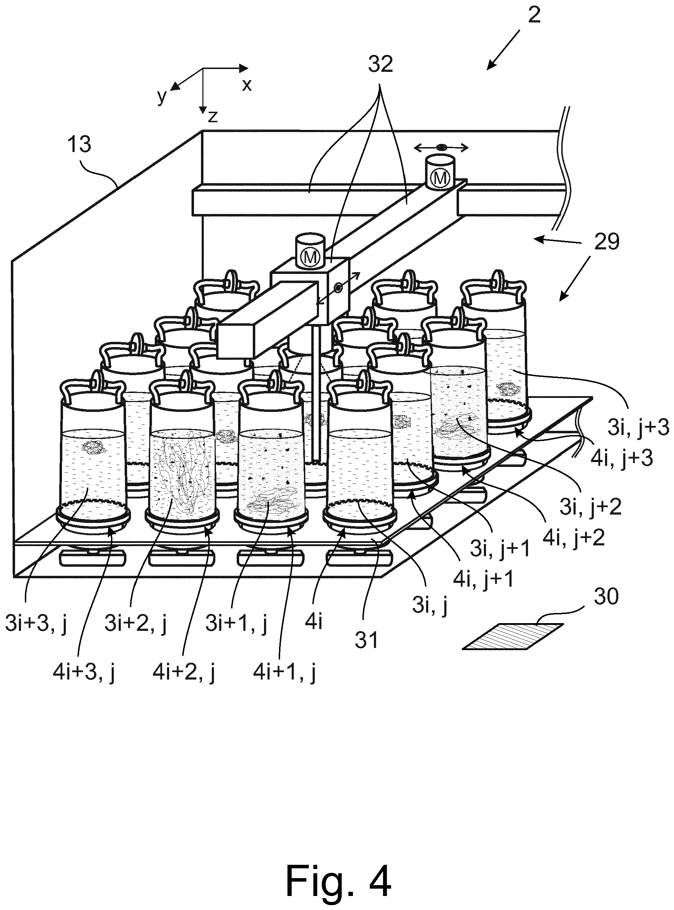

The proposed method for sterility testing is preferably performed using a sterility testing module 1 shown in Fig. 1 of a sterility testing system 2 shown in Fig. 4. The proposed method for sterility testing is based on optically analyzing at least one test liquid 3, which test liquid 3 is contained in a liquid container 4. Depending on the contamination state of the test liquid 3, non-liquid contaminants 5 are distributed in the test liquid 3. Fig. 3 shows on the left side examples of the distribution of the non-liquid contaminants 5 just as rough examples.

"Optical" means any kind of optical measurement in a physical sense, including but not limited to measurements based on visible light e.g. light scattering, light absorption, such as turbidity, the respective distribution of said turbidity, and/or based on non-visible light, such as UV or infra-red, or a combination thereof. Additionally, the optical measurement can be based on a pH shift visualised by the addition of at least one pH indicator to the test liquid 3, such as phenol- phthalein, bromthymol blue or methyl red.

The setup according to Fig. 1 comprises a control arrangement 6, which here and preferably comprises a local control unit 7 of the sterility testing module 1 and an external control unit 8, which may be a tablet, a personal computer or a server, which is arranged separately and remotely from, but in data connection with the local control unit 7. The data connection may be wire-based or, as indicated in Fig. 1, wireless.

It is essential, that in an analyzing routine 9 performed by the control arrangement 6, image-related data 10 representing at least one optical image of the test liquid 3, which have been generated by the sensor arrangement 11, are being transmitted from the sensor arrangement 11 to the control arrangement 6. Fig. 1 also shows, the sensor arrangement 11, which here and preferably is at least camera-based as will be explained later.

Here and preferably, the above-noted image-related data 10 are first being transferred to the local control unit 7, which may be a driver for the sensor arrangement 11 and which may also perform a preprocessing of the image-related data 10. The resulting data are then preferably transferred to the external control unit 8 for further processing. However, in order to achieve a compact overall structure, the complete analyzing routine 9 may be performed by the local control unit 7 in the vicinity of the sensor arrangement 11. In any case, the control arrangement 6 comprises computing hardware to perform the computations necessary to perform the analyzing routine 9.

According to the proposed method, in the analyzing routine 9 performed by the control arrangement 6, the contamination state of the test liquid 3 is then derived from the image-related data 10 based on the interrelation between the distribution characteristics of the contaminants 5 and the respective contamination state.

The term "distribution characteristics" is to be understood in a broad sense and refers to the entirety of possible spacial characteristics of contaminants 5, including but not limited to the distribution of contaminating organisms, particles, liquids, gases, ions, etc.

The test liquid 3 may well be the liquid, which sterility is primarily to be tested. However, here and preferably, the test liquid 3 is only analyzed in order to find out about the sterility of a sample liquid, which is different from the test liquid 3. In order to receive the test liquid 3 in the liquid container 4, a preparation routine is preferably performed. In a first step of this preparation routine, the sample liquid, which is primarily to be tested for sterility, is introduced via the inlet 4a at the top of the liquid container 4, is being passed through a filter 12 within the liquid container 4, and is being discharged via an outlet 4b at the bottom of the liquid container 4. Subsequently, the outlet 4b is being closed by a plug 4c. It is then preferred, that in a subsequent step of the preparation routine, the test liquid 3 in the form of a nutrient solution is introduced into the liquid container 4 via the inlet 4a. This may be done after the sample liquid has been discharged from the liquid container 4 through the filter 12 and, preferably, after a rinsing cycle. Then, the inlet 4a is being closed, here and preferably by connecting the inlet 4a to an air vent 4d of the liquid container 4. The filter 12 is preferably a membrane filter. However, other variants for the filter 12 are possible, depending on the field of application.

The above-noted concept for sterility testing is based on membrane filtration as noted above, which is only one preferred alternative. A second preferred alternative is the method for sterility testing based on direct inoculation. In this case, in the preparation routine, the test liquid 3 in the form of a combination of the sample liquid, which is primarily to be tested for sterility, and a nutrient solution is introduced into the liquid container 4 directly.

The test liquid 3 together with the filter 12 communicating with the test liquid 3 are being inserted into an incubation unit 13, which is only indicated in Fig. 4. After a predetermined incubation period, which may be 14 days, for example, the proposed analyzing routine 9 is preferably performed.

The distribution of the contaminants 5 in the test liquid 3 is in most cases represented by a certain turbidity 14, as well as by the distribution of the turbidity 14. In some cases, in addition, the distribution is represented by the occurrence of particle aggregates 15, 16. Additionally, the distribution of the contaminants 5 in the test liquid is represented by the detection and/or distribution of any solid, liquid or gaseous contaminant 5 based on measurements by laser or light beams. The frequency of the light on which the measurement is based, can be from the visible and/or non-visible spectrum, in particular from the UV or infra-red spectrum.

In the first case, the distribution of the contaminants 5 in the test liquid 3 is preferably represented by the intensity of turbidity 14 and/or the spreading of turbidity 14 and/or the geometric structure of turbidity 14 in the test liquid 3. In addition or as an alternative, as noted above, the distribution of the contaminants 5 in the test liquid 3 is represented by the occurrence of particle aggregates 15 and/or by the occurrence of sedimented particle aggregates 16. Depending on the sensor arrangement 11, at least part of those representations of the distribution of the contaminants 5 may be detected. In case of a camera-based sensor arrangement 11, there is a high potential for the detection of at least turbidity 14, as an area of turbidity is distinctive from an area without turbidity by contrast and/or color in the image of the test liquid 3.

In addition, in a prior analyzing routine preceding the above noted, image-related analyzing routine, prior property data representing at least one property of the test liquid 3 may be generated by the sensor arrangement 11. Preferably, the prior property data are being generated in the prior analyzing routine by at least one sensor of the sensor arrangement, which is/are different from the at least one sensor of the sensor arrangement, by which the image-related data are being generated.

The above noted, prior analyzing routine may contribute to a high reliabilty and/or high level of detail of the contamination state derived in the image-related analyzing routine. For this, the contamination state of the test liquid 3 is derived by the control arrangement 6 from the image-related data 10 and in addition from the prior property data based on the interrelation between the distribution characteristics of contaminants 5 in the test liquid 3 during the analyzing routine, the properties of the test liquid 3 during the prior analyzing routine and the respective contamination state.

In addition or as an alternative, the prior analyzing routine may serve to acquire an early indication of contamination. For this, during the prior analyzing routine, a preliminary contamination state of the test liquid 3 is derived by the control arrangement 6 from the prior property data based on the interrelation between properties of the test liquid 3 during the prior analyzing routine and the respective preliminary contamination state.

An example for an advantageous synopsis of the image-related data 10 during the image-related analyzing routine 9 and the prior property data during the prior analyzing routine will be given in the following for a contamination, which is based on the specific organism Staphylococcus aureus.

Staphylococcus aureus exhibits a golden-yellow color caused by staphyloxanthin, a carotinoid, which these bacteria produce as antioxidant. This color can be optically analyzed based on the generated image-related data, e.g. via photometrical analysis, light absorption and/or camera-based analysis. In the image-related analyzing routine 9, this property may be detected, wherein the derived preliminary contamination is generally "contaminated". However, it is not possible in this image-related analyzing routine 9 based on the optical image alone, to derive a contamination class representing the specific contaminating organism, since other organisms also produce carotinoids.

On the other hand, Staphylococcus aureus is able to produce lactic acid on anaerobic conditions. Lactic acid can be analyzed, for example, based on the generated prior property data, e.g. via a metabolite sensor measuring lactic acid. In the prior analyzing routine, this may lead to the derivation of a preliminary contamination state of the contamination class "contaminated". As the prior analyzing routine may be performed in an early stage preceding the image-related analyzing routine 9, the preliminary contamination state may be provided accordingly early, namely before the image-related analyzing routine 9 is completed. However, it is again not possible to derive a contamination class representing the specific contaminating organism, since other organisms also produce lactic acid.

During the image-related analyzing routine 9, by a correlation of both, image-related data and prior property data, it is possible to derive a contamination class representing the specific contaminating organism, since both features in synopsis unamigously identify the respective specific organism.

Just as a matter of completeness it may be pointed out, that the above noted lactic acid may also be detected during the image-related analyzing routine 9, as this image-related analyzing routine 9 is not necessarily restricted to image data in the narrow optical sense. In this case, the benefit of the early derivation of a preliminary contamination state would not be realized.

Additionally, identifying compounds may be added to the test liquid 3 before the prior analyzing routine and/or the analyzing routine 9 are initiated. Such identifying compound may further increase the level of accuracy in deriving a contamination class representing the specific contaminating organism. Exemplary identifying compounds may be proteins, in particular enzymes such as coagulase, oxidase or catalase, dyes such as N, N-Dimethyl-p-phenylendiammoniumchloride, indicators and/or ions,

The term "identifying compounds" refers to any physical, chemical or biological compound supporting the identification of specific organisms. As an example, Staphylococcus aureus produces the protein coagulase, which is able to react with fibrinogen, a glycoprotein. In case fibrinogen is added to a test liquid 3 containing Staphylococcus aureus, this reaction leads to a coagulation of fibrinogen exhibiting turbidity and aggregates, which are again optically analyzable, hence further supporting the identification of the specific organism.

There are various ways possible to initiate the prior analyzing routine. The easiest way is to have the user manually initiate the prior analyzing routine. However, the initiation of the prior analyzing routine may well be performed automatically, in particular, based on a trigger event. This trigger event may be the start, the middle and/or the end of the incubation period, which may easily be monitored by the control arrangement 6 based on time measurement. In addition, the prior analyzing routine may be initiated continuously and/or periodically during the incubation period, in order to take dynamic changes in the distribution of the contaminants 5 into account. Here and preferably, the prior analyzing routine is executed prior to the analyzing routine.

Further, there are various possible ways to define the contamination state of the test liquid 3. Here and preferably, the contamination state is defined such that it is represented by a contamination class out of a predefined group of contamination classes. Preferably, the preliminary contamination state derived in the prior analyzing routine as well as the contamination state derived in the image-related analyzing routine 9 are defined in the same way. In the analyzing routine 9, the contamination state is assigned one of the contamination classes.

In the easiest case, the predefined group of contamination classes only includes the contamination classes "contaminated" and "non-contaminated". As an alternative, the predefined group of contamination classes includes contamination classes each representing a specific organism such as Staphylococcus aureus, Bacillus subtilis, Pseudomonas aeruginosa or Kocuria rhizophila, Clostridium sporogenes or Bacteroides vulgatus, Candida albicans or Aspergillus niger.

These organisms, taken from the current version of the pharmacopeia, are just to be understood as an example. Further organisms, such as environmental isolates and/or such not listed in the pharmacopeia, are possible as well. Such contaminating organisms may come in homogeneous or heterogeneous groups. In case of a heterogeneous group the contamination state is represented by the contamination class, to which it essentially corresponds.

The distribution characteristics of the contamination class "Staphylococcus aureus" is mostly reflected by the occurrence of particle aggregates and/or by the occurrence of sedimented particles or particle aggregates. The distribution characteristics of the contamination class "Bacillus subtilis" is mostly reflected by the occurrence of particle aggregates and/or by the occurrence of a heterogeneous turbidity. The distribution characteristics of the contamination class "Pseudomonas aeruginosa or Kocuria rhizophila" is mostly reflected by the occurrence of a, preferably homogeneous, turbidity. The distribution characteristics of the contamination class "Clostridium sporogenes or Bacteroides vulgatus" is mostly reflected by the occurrence of a homogeneous turbidity. The distribution characteristics of the contamination class "Candida albicans" is mostly reflected by the occurrence of a particle film, in particular at the bottom of the liquid container. The distribution characteristics of the contamination class "Aspergillus niger" is mostly reflected by the occurrence of spatially confined particle clouds and/or by the occurrence of sedimented spatially confined particle clouds.

Looking at the examples for the distribution of the non-liquid contaminants 5 given on the left side of Fig. 3, it becomes apparent, that the contamination class of the contamination state may generally be derived from the image-related data 10 by simple image processing based on analysis criteria, which are assigned to the respective contamination class. Especially for a low number of contamination classes to be detected, this is an effective approach, which may be based on simple feature extraction. However, the more contamination classes are to be detected and the more variability of the distribution characteristics within one single contamination class is to be expected, this approach may lead to a very complex catalog of analysis criteria. In this case, the application of a machine learning mechanism appears to be a more powerful approach.

According to a preferred approach, the analyzing routine 9 is based on an above-noted machine learning mechanism 17, which is trained to derive the contamination state from the image-related data 10, in particular from the distribution characteristics of the contaminants 5 in the image-related data 10.

If the prior property data are included in the machine learning mechanism 17, the reliability and the level of detail may be enhanced during the image-related analyzing routine 9. Accordingly, it is preferably provided, that the image-related analyzing routine 9 is based on a machine learning mechanism 17, which is trained to derive the contamination state not only from the image-related data 10, in particular from the distribution characteristics of the contaminants 5 in the image-related data 10, but also from the prior property data.

Preferably, the machine learning mechanism 17 is based on a trained neural network. Particularly preferred is the application of a neural convolution network (CNN), as this has been proven to be very effective for analyzing images as noted above as well.

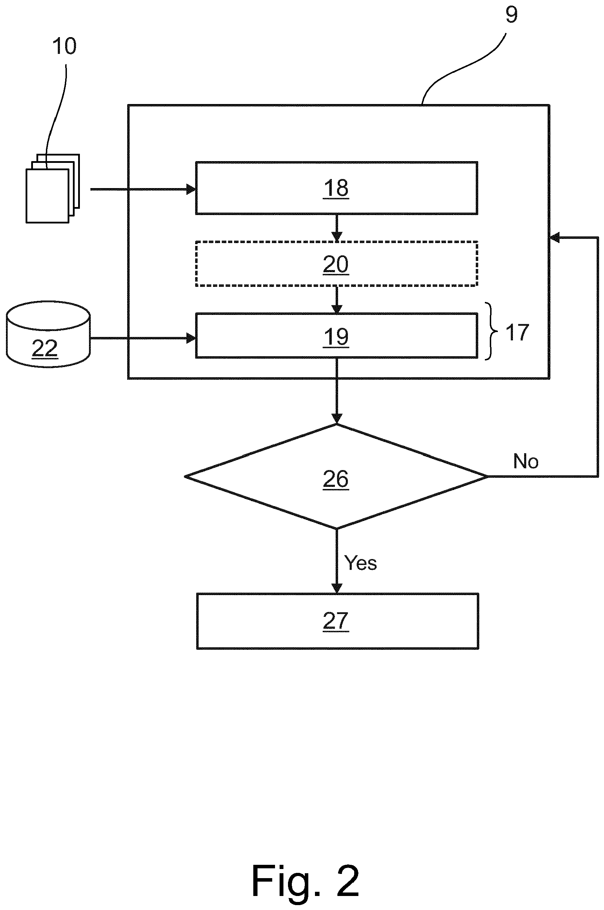

The analyzing routine 9 is shown in Fig. 2 as an example. After the transmittal of the image-related data 10 from the sensor arrangement 11 to the control arrangement 6 in an acquisition step 18, a classification step 19 is performed by the control arrangement 6, in which the contamination class of the contamination state is derived by the machine learning mechanism 17 from the image-related data 10. This is because the machine learning mechanism 17 has been trained to derive the contamination state from the image-related data 10 as noted above and as will be explained later.

In order to reduce the computation power necessary to perform the classification step 19, a crop step 20 may be provided directly after the image-related data 10 have been received from the sensor arrangement 11 in the acquisition step 18. In the crop step 20, a region of interest R is defined in the image-related data 10, which region of interest R is the subject of the classification step 19. In other words, the classification step 19 is only performed within the region of interest R, which reduces the complexity of the classification step 19. The region of interest R may well be a predefined area of the image represented by the image-related data 10. This is indicated for the image I_{1,n} in Fig. 3 as an example. With this, for example, unwanted optical reflections, which reflections might occur due to the individual geometrical structure of the sterility testing module 1, can systematically be disregarded.

As an alternative, the region of interest R may be defined by a user input. As another alternative, the region of interest R may automatically be defined by the control arrangement 6 based on image processing, for example, based on automatic feature extraction. Other alternatives for the definition of the region of interest R are possible.

As noted above, the machine learning mechanism 17 is being trained or has been trained in a training step 21. The concept of the training step 21 is shown in Fig. 3. According to this, the training step 21 is preferably based on annotated images I_{m,n} representing the contamination classes to be derived in the analyzing routine 9. On the left side of Fig. 3, the annotated images I_{m,n} are provided, which are each assigned an annotation A_{m,n}. This annotation A_{m,n} is being assigned to the respective image manually by a laboratory professional. Based on those annotated images I_{m,n}, a training data set 22 is generated by the control arrangement 6 or any other control system. In the analyzing routine 9, the classification step 19 is based on the training data set 22, as is indicated in Fig. 2.

The above-noted training step 21 is preferably performed by the external control unit 8 or any other control system, as for this step, considerable computation power is necessary. The resulting training data set 22 may then be downloaded into the respective part of the control arrangement 6, preferably into the local control unit 7, for performing the analyzing routine 9.

The sensor arrangement 11 is preferably at least camera-based and accordingly comprises a camera unit 23, which viewing direction C is towards the test liquid 3. The camera unit 23 may be a simple 2D camera unit, which even with high resolution is a low-cost component. For such a 2D camera unit, the viewing direction C is identical to the optical axis of the camera unit 23.

For the acquisition of the image-related data 10, the sensor arrangement 11 may provide an additional camera unit or a number of additional camera units with different viewing directions. In the following, to reduce complexity, only one camera unit 23 is discussed.

It may also be advantageous to provide the sensor arrangement 11 with a 3D camera unit, in order to include three-dimensional image-related data into the analyzing routine 9. However, somewhat three-dimensional image-related data may also be generated, if the focus of the camera unit 23 can be controlled by the control arrangement 6. This allows for acquiring image-related data 10 for images in different focal planes. As the above-noted turbidity 14 may be located at different heights with respect to the direction of gravity G and as it may be desirable to detect sedimented particle aggregations 16 which are mostly to be found at the bottom of the liquid container 4, at which the filter 12 is located, the acquisition of image-related data 10 for different focal planes 24a,24b,24c is particularly preferred. Three of those focal planes 24a,24b,24c are shown in Fig. 1 only as examples.

The viewing direction C of the camera unit 23 is preferably downwards or upwards with respect to gravity, wherein, preferably, the viewing direction C of the camera unit 23 deviates from the direction of gravity G by less than 10°.

Fig. 1 shows, that the liquid container 4 comprises a container body, which is preferably of a cup-like design, however with the top section and the bottom section being closed. In the bottom section, the above-noted filter 12 is located. The top section is transparent, such that the camera unit 23 may view through this top section. Moreover, the side sections of the liquid container 4 are transparent as well.

In an easy to realize embodiment, in the analyzing routine 9, the image-related data 10 of only one single image are being generated by the sensor arrangement 11. However, it may improve the classification result, if the derivation of the contamination state is performed based on image-related data 10, that represent a series of at least two images of the test liquid 3. The series of at least two images may regard images, that are offset from each other in a timewise manner or images, that are regarding different focal planes 24a,24b,24c.

It has been explained already, that the expression "image-related data" is to be understood in a broad sense, which means, that those image-related data may include the sensor data of other sensors of the sensor arrangement 11. Accordingly, here and preferably, the sensor arrangement 11 comprises at least one additional sensor, which provides additional sensor data, which contribute to the image-related data 10. With those additional sensors, sensor data regarding light scattering, light absorption, temperature, humidity, conductivity, bubbling speed, pH value, liquid level and/or sensor position may be acquired.

In another preferred embodiment, the sensor arrangement 11 comprises a light arrangement 25 for illuminating the test liquid 3. Here and preferably, the light arrangement 25 illuminates the test liquid 3 with the light of different wave lengths and/or different intensities. Those lighting parameters are preferably being controlled by the control arrangement 6.

Depending on the contamination state, which is checked in step 26 in view of predefined reaction criteria, it may be provided, that a reaction routine 27 is initiated by the control arrangement 6. Such reaction criteria may be directed to whether any contamination has been detected at all in the classification step 19. Preferably, the reaction routine 27 is then the output of an optical or an acoustic alert signal. It may also be advantageous, to create an electronic alert, which is either directly communicating the detection event to the user via a display or indirectly by sending the alert information to a central control system or the like.

There are various ways possible to initiate the analyzing routine 9. The easiest way is to have the user manually initiate the analyzing routine 9. However, the initiation of the analyzing routine 9 may well be performed automatically, in particular, based on a trigger event. This trigger event may be the end of the incubation period, which may easily be monitored by the control arrangement 6 based on time measurement. In addition, the analyzing routine 9 may be initiated continuously and/or periodically during the incubation period, in order to take dynamic changes in the distribution of the contaminants 5 into account.

Fig. 1 shows, that the sensor arrangement 11 and at least part of the control arrangement 6, here and preferably the local control unit 7, are being located on a single module carrier 28 of the sterility testing module 1. This is an advantage in view of compactness, but also in view of easy manufacturing, as both of the noted components may now be handled as one piece via the module carrier 28. According to the embodiment shown in Fig. 4, at least two test liquids 3_{¡,j} are provided, which are each contained in a separate liquid container 4_{i,j}. Preferably, for each test liquid 3_{i,j}, the above noted analyzing routine 9 is being performed. For this, the module carrier 28 is being moved relative to the liquid containers 4_{i,j}, as will be explained later.

In a particularly preferred embodiment, for at least two, in particular different, test liquids 3_{i,j}, which are each assigned to one and the same sample liquid, only one analyzing routine 9 is performed based on the image-related data 10 of those at least two different test liquids 3_{i,j}. This improves the specification performance as noted above. This is of particular interest, if the at least two test liquids 3i,j include different nutrient solutions in order to improve the classification result for the sample liquid. Here and preferably, the nutrient solutions used to prepare the respective test liquid 3i,j is one of the liquids listed for official sterility testings in the pharmacopoeia, preferably, that the sterility testing is fully compliant with the official regulations by the chapters US<71>; Ph. Eur 2.6.1 and/or JP 4.06 in the current, relevant version of the pharmacopoeia.

The pharmacopoeia represents the collected pharmaceutical rules for the quality, testing and storage of medicine and/or pharmaceuticals as well as the compounds, materials and methods used for their manufacturing. Here and preferably tryptic soy broth (TSB) media or fluid thioglycollate (FT) media are used as test liquids 3i,j. This redundancy of testings of at least two, in particular different, test liquids 3i,j, preferably nutrient solutions, which are each assigned to one and the same sample liquid, supports the identification of specific contaminants, in particular, if specific contaminants prefer specific, preferably also different, nutrient solutions.

As also shown in Fig. 4, here and preferably, a manipulation system 29 is provided, via which a relative movement between the liquid containers 4_{i,j} and at least part of the sensor arrangement 11 is being controlled by the control arrangement 6, such that for performing the analyzing routine 9, at least part of the sensor arrangement 11 is being relatively moved to the respective liquid container 4_{i,j}. Preferably, during relative movement between the liquid containers 4_{i,j} and at least part of the sensor arrangement 11 by the manipulation system 29, the sensor arrangement 11, here and preferably the camera unit 23, is at least partly performing a movement in the horizontal plane 30 with regard to gravity. With the camera unit 23 being directed downwards as explained earlier, all of the liquid containers 4_{i,j} and with it all of the test liquids 3_{¡,j} may be analyzed in separate analyzing routines 9 in an automated fashion. In addition or as an alternative, the camera unit 23 may be moved in three dimensions.

For this, a container carrier 31 is provided, in which the liquid containers 4_{i,j} are placed. Here and preferably, the liquid containers 4_{i,j} are arranged in a matrix along the horizontal plane 30, as shown in Fig. 4. The manipulation system 29 comprises a motorized manipulator 32 carrying at least part of the sensor arrangement 11, here and preferably the camera unit 23. The motorized manipulator 32 is being controlled by the control arrangement 6, such that for performing the analyzing routine 9, at least part of the sensor arrangement 11 is moved to the respective liquid container 4_{i,j}.

For maximum traceability of the analysis of the different test liquids 3_{i,j}, it is preferred, that the liquid container 3 comprises a barcode, which may be read by the camera unit 23 and transferred to the control arrangement 6. This double function of the camera unit 23 leads to a compact and cost-effective solution.

According to another teaching, a data storage device with a training data set 22 for use in the proposed method for sterility testing is claimed as such, wherein the training data set 22 is being produced by at least one training step 21 as noted above as well. All explanations regarding the first teaching are fully applicable.

According to another teaching, the above-noted control arrangement 6 as such for performing the proposed method for sterility testing is claimed. Again, all explanations regarding the first teaching are fully applicable.

According to another teaching, the above-noted sterility testing module 1 for performing the proposed method for sterility testing is claimed as such, which sterility testing module 1 comprises at least part of the sensor arrangement 11, preferably the camera unit 23, and at least part of the control arrangement 6, preferably the local control unit 7. All explanations regarding the first teaching are fully applicable regarding this additional teaching as well.

According to another teaching, the above-noted sterility testing system 2 with the above-noted sterility testing module 1 and with a container carrier 31 for carrying at least two liquid containers 4_{i,j} for test liquids 3_{¡,j} is claimed as such. All explanations regarding the first teaching are fully applicable.

Preferably the sterility testing system 2 comprises an above-noted manipulation system 29, via which a relative movement between a number of liquid containers 4_{i,j} and at least part of the sensor arrangement 11 is controlled by the control arrangement 6, such that for performing the analyzing routine 9, at least part of the sensor arrangement 11 may be relatively moved to the respective liquid container 4_{i,j}.

Further preferably, the sterility testing system 2 comprises an above-noted container carrier 31, in which the liquid containers 4_{i,j} may be placed. Here and preferably, the liquid containers 4_{i,j} are arranged in a matrix along the horizontal plane 30, as shown in Fig. 4. The manipulation system 29 comprises a motorized manipulator 32 carrying at least part of the sensor arrangement 11, which manipulator 32 is being controlled by the control arrangement 6, such that for performing the analyzing routine 9, at least part of the sensor arrangement 11 is moved to the respective liquid container 4_{i,j}.

Further preferably, the manipulation system 2 is designed such that during relative movement between the liquid containers 4_{i,j} and at least part of the sensor arrangement 11 by the manipulation system 29, the sensor arrangement 11, in particular the camera unit 23, is at least partly performing a movement in the horizontal plane 30 with regard to gravity and/or in three dimensions. As shown in Fig. 4, the manipulator 32 of the manipulation system 29 is designed in the form of an XY-table, which makes the realization cost-effective based on the use of standardized mechanical components.

Generally, the manipulation system 29 may also be realized in other constructional ways. For example, the manipulation system 29 may be structured as a carousel system, such that the liquid containers 4_{i,j}. are arranged along an arc of a circle or along a circle. Other arrangements are possible.

## Claims

1. Method for sterility testing based on optically analyzing at least one test liquid (3), which test liquid (3) is contained in a liquid container (4),
**characterized in**
**that** in an analyzing routine (9), image-related data (10) representing at least one optical image (I) of the test liquid (3) are being generated by a sensor arrangement (11) and that the contamination state of the test liquid (3) is derived by a control arrangement (6) from the image-related data (10) based on the interrelation between the distribution characteristics of contaminants (5) in the test liquid and the respective contamination state.

2. Method according to claim 1, **characterized in that** preceding the analyzing routine (9), in order to receive the test liquid (3) in the liquid container (4), a preparation routine is performed, preferably, that in a first step of the preparation routine, a sample liquid to be tested for sterility is being passed through a filter (12), in particular a membrane filter, which filter (12) is located within the liquid container (4), and that in a subsequent step of the preparation routine, the test liquid (3) in the form of a nutrient solution is introduced into the liquid container (4), or, that in the preparation routine, the test liquid (3) in the form of a combination of the sample liquid to be tested for sterility and a nutrient solution is introduced into the liquid container (4).

3. Method according to claim 1 or 2, **characterized in that** the distribution of the contaminants (5) in the test liquid (3), which is detectable by the sensor arrangement (11), is represented by the intensity of turbidity (14) and/or the spreading of turbidity (14) and/or the geometric structure of turbidity (14) in the test liquid (3), and/or, that the distribution of the contaminants (5) in the test liquid (3) is represented by the occurrence of particle aggregates (15) and/or by the occurrence of sedimented particle aggregates (16).

4. Method according to any one of the preceding claims, **characterized in that** in a prior analyzing routine preceding the analyzing routine (9), prior property data representing at least one property of the test liquid (3) are being generated by the sensor arrangement (11).

5. Method according to claim 4, **characterized in that** the prior property data are being generated in the prior analyzing routine by at least one sensor of the sensor arrangement (11), which is/are different from the at least one sensor of the sensor arrangement, by which the image-related data are being generated.

6. Method according to claim 4 or 5, **characterized in that** the contamination state of the test liquid (3) is derived by the control arrangement (6) from the image-related data (10) and the prior property data based on the interrelation between the distribution characteristics of contaminants (5) in the test liquid (3) during the analyzing routine (9), the properties of the test liquid (3) during the prior analyzing routine and the respective contamination state.

7. Method according to any one of the claims 4 to 6, **characterized in that** during the prior analyzing routine, a preliminary contamination state of the test liquid (3) is derived by the control arrangement (6) from the prior property data based on the interrelation between properties of the test liquid (3) during the prior analyzing routine and the respective preliminary contamination state.

8. Method according to any one of the preceding claims, **characterized in that** the contamination state is represented by a contamination class out of a predefined group of contamination classes and that in the analyzing routine (9), the contamination state is assigned one of the said contamination classes, preferably, that the predefined group of contamination classes only includes the contamination classes "contaminated" and "non-contaminated", or, that the predefined group of contamination classes includes contamination classes each representing a specific organism such as Staphylococcus aureus, Bacillus subtilis, Pseudomonas aeruginosa or Kocuria rhizophila, Clostridium sporogenes or Bacteroides vulgatus, Candida albicans or Aspergillus niger.

9. Method according to any one of the preceding claims, **characterized in that** in the analyzing routine (9), the contamination class of the contamination state is derived from the image-related data (10) by image processing based on analysis criteria, which are assigned to the respective contamination class.

10. Method according to any one of the preceding claims, **characterized in that** the analyzing routine (9) is based on a machine learning mechanism (17), which is trained to derive the contamination state from the image-related data (10), in particular from the distribution characteristics of the contaminants (5) in the image-related data (10).

11. Method according to any one of the preceding claims, **characterized in that** the analyzing routine (9) is based on a machine learning mechanism (17), which is trained to derive the contamination state from the image-related data (10), in particular from the distribution characteristics of the contaminants (5) in the image-related data (10), and from the prior property data.

12. Method according to claim 10 or 11, **characterized in that** the machine learning mechanism (17) is based on a trained neural network, preferably on a neural convolution network (CNN).

13. Method according to any one of the claims 10 to 12, **characterized in that** the analyzing routine (9) comprises a classification step (19), in which the contamination class of the contamination state is derived by the machine learning mechanism (17) from the image-related data (10).

14. Method according to claim 13, **characterized in that** in a crop step (20), a region of interest (R) is defined in the image-related data (10), which region of interest (R) is subject of the classification step (19), preferably, that the region of interest (R) is a predefined area of the image (I) represented by the image-related data (10) or that the region of interest (R) is defined by a user input or that the region of interest (R) is automatically defined by the control arrangement (6) based on image processing, preferably based on automatic feature extraction.

15. Method according to any one of the claims 10 to 14, **characterized in that** the machine learning mechanism (17) is being trained or has been trained in a training step (21), preferably based on annotated images (I_{m,n}) representing the contamination classes to be derived in the analyzing routine (9), further preferably, that in the training step (21), a training data set (22) is generated by the control arrangement (6), wherein the classification step (19) in the analyzing routine (9) is based on the training data set (22).

16. Method according to any one of the preceding claims, **characterized in that** the sensor arrangement (11) includes a camera unit (23), which is directed towards the test liquid (3), preferably, that the focus of the camera unit (23) may be controlled by the control arrangement (6).

17. Method according to any one of the preceding claims, **characterized in that** the viewing direction (C) of the camera unit (23) is downwards or upwards with respect to gravity, preferably, that the viewing direction (C) of the camera unit (23) deviates from the direction of gravity (G) by less than 10º.

18. Method according to any one of the preceding claims, **characterized in that** in the analyzing routine (9), the derivation of the contamination state is performed based on image-related data (10), that represent a series of at least two images (I) of the test liquid (3).

19. Method according to any one of the preceding claims, **characterized in that** the sensor arrangement (11) comprises a light arrangement (25) for illuminating the test liquid (3), preferably, that the light arrangement (25) illuminates the test liquid (3) with the light of different wave lengths and/or different intensities, preferably, that the light arrangement (25) is being controlled by the control arrangement (6).

20. Method according to any one of the preceding claims, **characterized in that** at least two test liquids (3_{i,j}) are provided, which are each contained in a separate liquid container (4_{i,j}), preferably, that for each test liquid (3_{,j}), the analyzing routine (9) is being performed.

21. Method according to any one of the preceding claims, **characterized in that** for at least two, in particular different, test liquids (3_{i,j}), which are each assigned to one and the same sample liquid, only one analyzing routine (9) is performed based on the image-related data (10) relating to those at least two different test liquids (3_{i,j}).

22. Method according to claim 20 and, if so, to claim 21, **characterized in that** a manipulation system (29) is provided, via which a relative movement between the liquid containers (3_{i,j}) and at least part of the sensor arrangement (11) is being controlled by the control arrangement (6), such that for performing the analyzing routine (9), at least part of the sensor arrangement (11) is being relatively moved to the respective liquid container (3_{i,j}), preferably, that during relative movement between the liquid containers (3_{i,j}) and at least part of the sensor arrangement (11) by the manipulation system (29), the sensor arrangement (11), in particular the camera unit (23), is at least partly performing a movement in the horizontal plane (30) with regard to gravity.

23. Method according to claim 22, **characterized in that** a container carrier (31) is provided, in which the liquid containers (3_{i,j}) are placed and that the manipulation system (29) comprises a motorized manipulator (32) carrying at least part of the sensor arrangement (11), which manipulator (32) is being controlled by the control arrangement (6), such that for performing the analyzing routine (9), at least part of the sensor arrangement (11) is moved to the respective liquid container.

24. Data storage device with a training data set (22) for use in a method according to any one of the preceding claims, being produced by at least one training step (21) according to claim 15.

25. Control arrangement for performing the method according to any one of the claims 1 to 23.

26. Sterility testing module comprising at least part of a sensor arrangement (11) and at least part of a control arrangement (6) for performing the method according to any one of the claims 1 to 23.

27. Sterility testing system with a sterility testing module (1) according to claim 26 and a container carrier (31) for carrying at least two liquid containers (4_{i,j}) for test liquids (3_{i,j}).

28. Sterility testing system according to claim 27, **characterized in that** it comprises a manipulation system (29), via which a relative movement between the liquid containers (4_{i,j}) and at least part of the sensor arrangement (11) is controlled by the control arrangement (6), such that for performing the analyzing routine (9), at least part of the sensor arrangement (11) may be relatively moved to the respective liquid container (4_{i,j}).

29. Sterility testing system according to claim 28, **characterized in that** the sterility testing system (2) comprises a container carrier (31), in which the liquid containers (4_{i,j}) for test liquids (3_{i,j}) may be placed and that the manipulation system (29) comprises a motorized manipulator (32) carrying at least part of the sensor arrangement (11), which manipulator (32) is being controlled by the control arrangement (6), such that for performing the analyzing routine (9), at least part of the sensor arrangement (11) is moved to the respective liquid container (4i,j).

30. Sterility testing system according to any one of the claims 27 to 29, **characterized in that** the manipulation system (29) is designed such that during relative movement between the liquid containers (4_{i,j}) and at least part of the sensor arrangement (11) by the manipulation system (29), the sensor arrangement (11), in particular the camera unit (23), is at least partly performing a movement in the horizontal plane (30) with regard to gravity and/or in three dimensions.
